# EUROPEAN PATENT SPECIFICATION

(11) **EP 1 432 822 B1**
(45) Date of publication and mention of the grant of the patent: **23.05.2007**
(21) Application number: 02766349.1
(22) Date of filing: 10.09.2002
(51) Int. Cl.: C12Q 1/68, C12P 19/34, C12N 15/63, C07K 1/00, C07H 21/04, C07K 1/14

(54) **METHOD FOR RECOVERING AND ANALYZING A CELLULAR COMPONENT OF CULTURED CELLS WITHOUT HAVING TO HARVEST THE CELLS FIRST**
VERFAHREN ZUR GEWINNUNG UND ANALYSE EINES ZELLBESTANDTEILS AUS KULTIVIERTEN ZELLEN OHNE DIE NOTWENDIGKEIT FÜR EIN VORHERIGES ERNTEN DER ZELLEN
PROCEDE DE RECUPERATION ET D'ANALYSE D'UNE COMPOSANTE CELLULAIRE DE CELLULES CULTIVEES SANS BESOIN DE RECOLTER LES CELLULES AU PREALABLE

(30) Priority: 10.09.2001 US 323146 P
(43) Date of publication of application: 30.06.2004
(73) Proprietor: EMD Biosciences, Inc., San Diego, CA 92121 (US)
(72) Inventor: GRABSKI, Anthony, C., Blue Mounds, WI 53517 (US)
(74) Representative: Duckworth, Timothy John
(86) International application number: PCT/US2002/030288
(87) International publication number: WO 2003/023050

(56) References cited:
- US-A- 5 240 834
- CHOMCZYNSKI P ET AL: "SINGLE-STEP METHOD OF RNA ISOLATION BY ACID GUANIDINIUM THIOCYNATE-PHENOL-CHLOROFORM EXTRACTION" ANALYTICAL BIOCHEMISTRY, ACADEMIC PRESS, NEW YORK, NY, US, vol. 162, 1987, pages 156-159, XP000972553 ISSN: 0003-2697
- RAMIREZ-SOLIS R ET AL: "GENOMIC DNA MICROEXTRACTION: A METHOD TO SCREEN NUMEROUS SAMPLES1" ANALYTICAL BIOCHEMISTRY, ACADEMIC PRESS, SAN DIEGO, CA, US, vol. 201, no. 2, 1 March 1992 (1992-03-01), pages 331-335, XP000257967 ISSN: 0003-2697
- TAGLIAFERRO ET AL.: 'Use of a rapid and simple method to extract provial DNA in the identification of HIV-1 by PCR' MICROBIOLOGICA vol. 18, 1995, pages 303 - 306, XP002961240
- BOADO ET AL.: 'A one-step procedure for isolation of poly(A)+ mRNA from isolated brain capillaries and endothelial cells in culture' JOURNAL OF NEUROCHEMISTRY vol. 57, no. 6, 1991, pages 2136 - 2139, XP002961241
- HAELEWYN ET AL.: 'A rapid single-step purification method for human interferon-gamma from isolated escherichia coli inclusion bodies' BIOCHEMISTRY AND MOLECULAR BIOLOGY INTERNATIONAL vol. 37, no. 6, December 1995, pages 1163 - 1171, XP008014369
- SUMMERTON ET AL.: 'A rapid method for preparation of bacterial plasmids' ANALYTICAL BIOCHEMISTRY vol. 133, 1983, pages 79 - 84, XP002120178

## Description

### BACKGROUND OF THE INVENTION

Existing methods for recovering cellular components such as proteins and nucleic acids require an initial cell harvest step, which concentrates cell mass and removes media components. For example, many expression vectors have been developed to express a target protein in cultured cells. To produce and purify the target protein, traditional protein isolation technology usually begins with culturing the cells containing an expression vector for the target protein in liquid media under conditions for maximum target protein expression. Cells containing the expressed protein are harvested by centrifugation or filtration, resuspended in a buffer or lysis reagent, mechanically or chemically disrupted to prepare the cell extract, and finally the cellular components are fractionated through multiple mechanical, chemical, and biochemical processing procedures (1-4). Centrifugation and mechanical lysis steps are difficult to automate and miniaturize for the purpose of purifying small amounts of many proteins in parallel.

### BRIEF SUMMARY OF THE INVENTION

The present invention provides a method for recovering a protein from cultured cells comprising:
a) forming a lysis reaction by adding a cell lysis reagent to a cell culture medium without harvesting the cells wherein the cell lysis reagent is a detergent, enzyme, glycoside, or mixture thereof;
b) adding a solid matrix to the lysis reaction to form a mixture, wherein an affinity or adsorptive ligand is attached to the solid matrix and the solid matrix absorbs a protein from the mixture; and
c) separating the solid matrix from the mixture.

The present invention may therefore be used to provide a method for recovering and analyzing a cellular component of cultured cells without harvesting the cells.

It is a feature of the present invention that the cells are lysed with a non-mechanical method.

It is an advantage of the present invention that the procedure of recovering and analyzing a cellular component is easier to be performed in a high throughout manner.

Further objects, features and advantages of the present invention will be apparent from the following detailed description when taken in conjunction with the accompanying claims and drawings.

### BRIEF DESCRIPTION OF THE SEVERAL VIEWS OF THE DRAWINGS

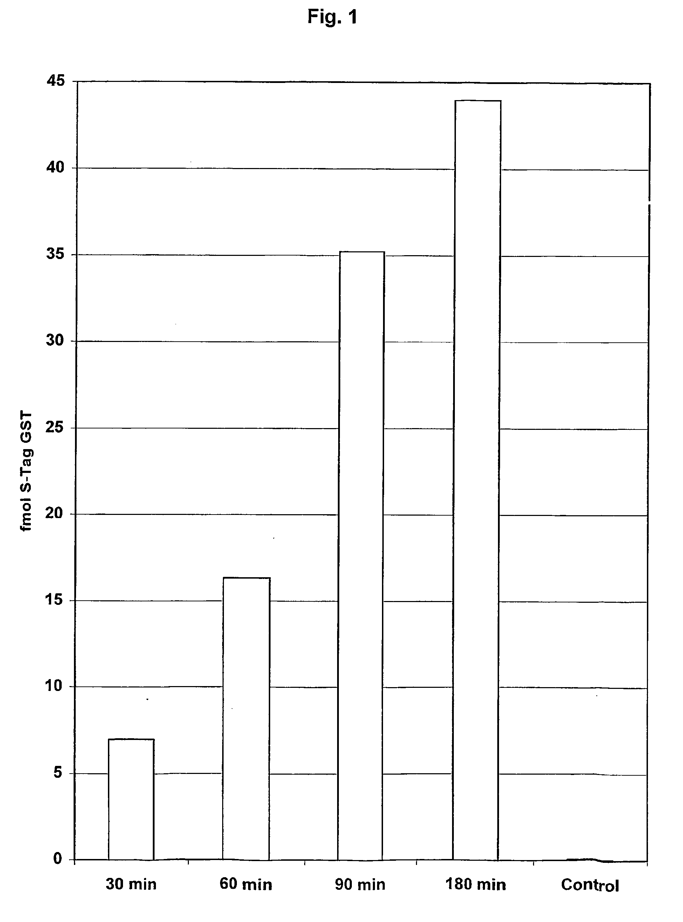

Fig. 1 shows time course of induction of S•Tag GST with FRETWorks S•Tag assay.

### DETAILED DESCRIPTION OF THE INVENTION

The conventional methods of recovering or analyzing a cellular component of cultured cells require generating a cell extract of the cultured cells by harvesting the cells and lysing the harvested cells. It is disclosed here that a cellular component can be recovered and analyzed by lysing the cells directly in the liquid culture medium without harvesting the cells. The component released from the cells can be recovered from and analyzed in the medium directly. The elimination of the cell-harvest step makes it possible for the recovery and analysis procedures to be conducted in a high throughput fashion. As an illustration, the examples below show that cellular proteins are successfully recovered and the activity and quantity of which are successfully measured in a cell extract obtained by lysing the cells directly in the culture medium. In addition, a high throughput adaptation of a protein recovery procedure is also shown.

In one embodiment, the present invention is a method of producing a cell extract suitable for recovery and analysis of a cellular component without having to harvest the cells. The method involves adding a cell lysis reagent into the culture medium to lyse the cells. Examples of cellular components that can be analyzed this way include proteins. By analyzing a cellular component, we mean quantifying the amount or measuring the activity of the cellular component. The activity of a cellular component can be enzymatic activities, binding activities or other biological activities.

Both prokaryotic and eukaryotic cells can be lysed as described above to recover or analyze a cellular component therein. It does not matter whether the cells are suspended in the medium or adhere to the wall of a cultureware. Bacterial cells such as *E*. *coli* cells and certain eukaryotic cells such as insect cells and yeast cells are hard to lyse and lysing these cells with a detergent requires reagents with relatively high detergency. However, as demonstrated in the examples below, adding detergent-based reagents that can break up these cells in the medium does not prevent successful recovery and analysis of a protein produced by the cells.

There are many reagents that can be used in the present invention to lyse cultured cells in the medium. Examples of these reagents include but are not limited to detergents, enzymes such as lysozymes, chitinases, or glucanases, glycosides and a mixture thereof. Preferably, a detergent-based reagent is used in the present invention. The reagent that is added into the medium can be either in solution form or in powder form.

Multiple agents can be combined for optimal cell lysis efficacy. For example, lysozyme and one or more detergents can be used together to lyse bacterial cells. The detergent(s) disrupt the cell membrane and the lysozyme hydrolyses the cell wall. If the subsequent application does not involve analyzing nucleic acids from the cells, a nuclease can be added into the medium to reduce the viscosity of the cell extract. The reduction in viscosity facilitates downstream processes such as protein purification and assays especially in high throughput applications.

In another embodiment, the present invention is a method of recovering a protein from cultured cells by lysing the cells as described above and capturing and isolating the protein from the rest of the medium through affinity adsorption. Certain proteins are secreted into the medium during culture and certain cells lyse during culture releasing the content into the medium. The protein recovery method of the present invention allows recovery of these proteins that the conventional method involving harvesting cells will lose.

To capture a target protein from the medium after the cells have been lysed, a solid matrix that can adsorb the target protein is added into the medium to form a protein-matrix complex. The complex is then separated from the rest of the medium and preferably washed, and the target protein is subsequently separated from the matrix. The separation and washing steps can be conducted in the same cultureware where the cells were cultured and lysed or the medium containing the protein-matrix complex can be poured into a holder to form a column for washing and eluting the target protein. Examples of each are described in the examples below.

Alternatively, one can pre-make a column of a solid matrix that can adsorb the target protein and pour the medium containing the lysed cells through the column. The protein is retained in the column by forming a protein-matrix complex. The protein-matrix complex is preferably washed and the protein can be subsequently eluted from the column.

If the target protein to be recovered is not soluble in the medium, one can use a filter to separate the soluble and insoluble fractions of the medium. The insoluble protein retained by the filter can be solubilized using a suitable solution and the solution containing the protein can be treated the same way as the medium is treated (described above) to recover the protein.

It is well within the capability of a skilled artisan to select or make a solid matrix for capturing a target protein based on the nature and characteristics of the target protein. For example, if the target protein is His-tagged, HisBind resin (Novagen, Inc., Madison, WI) can be used. Depending on the target protein to be recovered, other capture matrix that may be useful include but are not limited to solid supports or magnetic particles attached by an affinity or adsorptive ligand such as Ni-NTA His-Bind® (Novagen, Inc., Madison, WI), GST, S-Protein, antibodies, and charged fimctionalities.

By way of example, but not limitation, examples of the present invention are described below.

### EXAMPLE 1

### Extraction and Purification of Proteins from E. coli without Harvesting Cells

### Methods

General protocol for PopCulture^{™} (Novagen, Inc., Madison, WI) extraction and purification: Cells were cultured in liquid media under conditions for target protein production. 0.1 culture volume PopCulture^{™} Reagent was added, mixed, and incubated for 10 minutes at room temperature. Optionally, lysozyme and/or Benzonase® Nuclease (Novagen, Inc., Madison, WI) were added, mixed and incubated for 10-15 minutes at room temperature. Equilibrated affinity resin was added, mixed, and incubated for 5 minutes at room temperature. The affinity resin was separated from the culture extract by filtration or magnetic isolation. The affinity resin was washed. The target protein was eluted using the appropriate elution buffer. The affinity resin was removed. The purified protein was analyzed.

IMAC purification of a His·Tag fusion protein from *E*. *coli* total culture extracts (batch/column purification): *E*. *coli* strain BL21(DE3) containing pET-41b(+) was grown in liquid culture and protein expression induced with I mM IPTG for approximately 3 h (final OD600 = 9.0). Samples (2.7 ml) of the culture were dispensed into 15-ml tubes and 0.3 ml PopCulture Reagent was added to each tube (except for the control). The 2.7-ml control sample was centrifuged at 10,000 x g for 5 min to harvest the cells, and the supernatant removed and discarded. The cell pellet from the control was suspended in 0.3 ml BugBuster Reagent (Novagen, Inc., Madison, WI). All samples were incubated for 10 min at room temperature, treated with 2 µl Benzonase Nuclease, and processed. Target proteins were eluted with 2 x 150 µl of 0.5X His·Bind Elute Buffer.

IMAC purification of a His·Tag fusion protein from *E. coli* total culture extracts (magnetic purification): The same recombinant used above for the batch/column purification was induced with IPTG for 3 h (final OD600 = 4.8). The culture was dispensed in 1.0 ml samples into a deep 96-well plate (2 ml well capacity) and 0.1 volume PopCulture Reagent was added per well. After pipetting up and down to mix, 1 µl Benzonase was added followed by another mixing step and the samples were incubated 10 min at room temperature. His·Bind Magnetic Agarose Beads (Novagen, Inc., Madison, WI) (50 µl of a 50% slurry equilibrated in 1X His·Bind Binding Buffer (Novagen, Inc., Madison, WI)) were added to each sample, mixed, and incubated 5 min at room temperature. The samples were subjected to a magnetic field using pin magnets to collect the beads. The beads were washed three times with 750 µl His·Bind Wash Buffer (Novagen, Inc., Madison, WI). Target protein was eluted with 200 µl 0.5X His·Bind Elute Buffer (Novagen, Inc., Madison, WI) followed by 100 µl 0.5X His·Bind Elute Buffer. All samples were analyzed by SDS PAGE (4-20% gradient gels) and Coomassie blue staining.

GST·Bind purification of a GST fusion protein from *E. coli* total culture extracts (batch/column purification): *E. coli* strain BL21(DE3) containing pET-41b(+) was grown in liquid culture and protein expression induced with 1 mM IPTG for approximately 3 h (final OD600 = 2.1). Samples (3 ml) of the culture were dispensed into 15-ml tubes and 0.3 ml PopCulture Reagent was added to each tube (except for the control). The 30-ml control sample was centrifuged at 10,000 x g for 5 min to harvest the cells, and the supernatant removed and discarded. The cell pellet from the control was suspended in 1X BugBuster Reagent at a ratio of 5 ml/g cells. All samples were incubated for 10 min at room temperature, treated with 2 µl Benzonase Nuclease, and processed. Target proteins were eluted with 2 x 375 µl of GST Elute Buffer (Novagen, Inc., Madison, WI).

GST·Bind purification of a GST fusion protein from *E*. *coli* total culture extracts (magnetic purification): The same recombinant used above for batch/column purification was induced with IPTG for 3 h (final OD600 = 4.8). The culture was processed exactly as described above for the magnetic purification of His·Tag fusion protein (n = 8 wells), except that GST·Bind Magnetic Agarose Beads (Novagen, Inc., Madison, WI) and 1X GST Bind/Wash Buffer (Novagen, Inc., Madison, WI) were used for purification. Target protein was eluted with 100 µl 1X GST Elute Buffer (Novagen, Inc., Madison, WI). All samples were analyzed by SDS PAGE (4-20% gradient gels) and Coomassie blue staining.

Effect of lysozyme on PopCulture extraction efficiency: *E*. *coli* strain BL21 (DE3) containing pET-28b(+) β-galactosidase or pET-41b(+) was grown in liquid culture and protein expression induced with 1 mM IPTG for approximately 3 h. To obtain sufficient protein for gel analysis, cells were concentrated prior to treatment, and were resuspended in a 1:10 dilution of PopCulture Reagent and incubated 10 min at room temperature. The indicated samples received an additional 15 min treatment with chicken egg lysozyme or recombinant lysozyme.

Effect of lysozyme on PopCulture extraction efficiency (comparing BL21 (DE3) and BL21 (DE3)pLysS hosts): BL21 (DE3) and BL21 (DE3)pLysS hosts containing pET β-galactosidase recombinants were grown in liquid culture and protein expression induced with 1 mM IPTG for approximately 3 h. Samples of the cultures were processed as described above for the BL21 (DE3) host. Total cell protein (TCP) samples were prepared by resuspending cell pellets in SDS sample buffer. The TCP and equal volumes of all PopCulture extracts were analyzed by SDS PAGE (4-20% gradient gels) and Coomassie blue staining.

### Results:

Purification of a His·Tag fusion protein from *E*. *coli* total culture extracts: As a test vector for *E*. *coli* extraction and purification we used pET-41b(+), which ex-presses a 35.6 kDa GST•Tag^{™}/ His·Tag^{®} fusion protein that can be purified using immobilized metal chelation chromatography (IMAC; His•Bind^{®} Resins (Novagen, Inc., Madison, WI)) or immobilized glutathione (GST•Bind^{™} Resins (Novagen, Inc., Madison, WI)). Both affinity purification methods are compatible with the conditions of total culture extraction with the PopCulture Reagent, and magnetic formats are available that are well suited for high throughput applications.

For testing IMAC purification, the general protocol was used with three different affinity supports: His·Bind Resin, Ni-NTA His·Bind Resin, and His·Bind Magnetic Agarose Beads. The His·Bind Resin was pre-charged with Ni²⁺ before equilibration with 1X His·Bind Buffer, and the other two supports (which are already Ni²⁺-charged) were directly equilibrated in the same buffer. With His·Bind and Ni-NTA His·Bind samples, the target protein was captured in batch mode and then the resins were transferred to small columns for final washing and elution steps. Multiwell filter plates can also be used for this application. With His·Bind Magnetic Agarose Beads, the entire purification procedure was performed in batch mode using 2-ml deep 96-well plates and a magnetic pin rack to collect the beads for binding, wash and elution steps. As a control, cells were harvested by centrifugation from an equal volume of culture, and protein was extracted with BugBuster^{™} Reagent. The control extract was clarified by centrifugation and the target protein purified using His·Bind Resin.

The results are summarized Table 1. The data show that with all three types of IMAC matrix, the yield of purified target protein recovered from PopCulture total culture extracts was significantly greater than the yield from the control purification. Furthermore, the purity of the target protein was similar to that of protein purified by the standard method using centrifugation for cell harvest and extract clarification. (Several minor truncated GST products are routinely observed.) Most notably, the use of His·Bind Magnetic Agarose Beads enabled the entire procedure to be carried out in a single tube without the need for columns or centrifugation.

**Table 1. Purification of His•Tag ® GST expressed in E. coli**

| Purification Method | Yield¹ | Purity ² |
|---|---|---|
| Standard His•Bind ® 74 83 | 74 | 83 |
| PopCulture^{™} His•Bind | 111 | 83 |
| PopCulture Ni-NTA His•Bind ³ | 170 | 85 |
| PopCulture His•Bind Magnetic ³ | 128 | 94 |
| Standard GST•Bind^{™} 42 92 | 42 | 92 |
| PopCulture GST•Bind 45 90 | 45 | 90 |
| PopCulture GST•Bind Magnetic³ | 40 | 94 |

| | | |
|---|---|---|
| 1. Yield in micrograms of target protein purified per ml of culture, as determined by BCA protein assay. 2. % purity determined by scanning densitometry of Coomassie blue stained SDS polyacrylamide gels. 3. Data represent the average of 8 separate wells processed in parallel. | | |

Purification of a GST fusion protein from total culture extracts: The GST•Tag/His•Tag fusion protein expressed from pET-41b(+) was also purified with GST·Bind Resin, using the affinity of the GST (glutathione-S-transferase) domain for immobilized reduced glutathione on the resin. As in the His·Bind purification experiments, two different GST·Bind formats were used. Table 1 show the results of these purifications. A batch protocol was performed with the standard GST·Bind Resin, and a magnetic protocol was used with GST·Bind Magnetic Agarose Beads. Extraction and purification of this protein from PopCulture total culture extracts using the GST affinity produced yields and purity similar to the controls using standard harvest and extraction procedures.

Effect of lysozyme: Lysozyme, which cleaves a bond in the peptidoglycan layer of the *E. coli* cell wall, is widely used to enhance cell lysis. We therefore investigated the effect of lysozyme on the efficiency of protein extraction when used in combination with the PopCulture Reagent. Table 2 demonstrates that lysozyme increased the yield of proteins in PopCulture total extracts. In Table 2, BL21(DE3) and BL21(DE3)pLysS hosts were used for expression and purification of a His·Tag β-galactosidase fusion protein en-coded by a pET plasmid. Parallel cultures were processed with PopCulture Reagent, either omitting or including the addition of lysozyme to the procedure. The data show that the yield of this large protein (a tetramer composed of 118 kDa subunits) was increased two- to three-fold by including a source of lysozyme in the extraction. Furthermore, extraction was equally effective using a pLysS host (which expresses low levels of T7 lysozyme), or adding purified chicken egg or recombinant lysozyme to the PopCulture extraction with a non-pLysS host.

**Table 2. Effect of lysozyme on PopCulture yield of His·Tag β-gal**

| Host | Cell Mass ¹ | Lys ² | Yield ³ | Purity ⁴ |
|---|---|---|---|---|
| BL21(DE3)pLysS | 11 | -- | 27 | 94 |
| BL21(DE3)pLysS | 11 | chicken | 23 | 94 |
| BL21(DE3)pLysS | 11 | recomb. | 26 | 87 |
| BL21(DE3) | 15 | -- | 11 | 84 |
| BL21(DE3) | 15 | chicken | 38 | 93 |
| BL21(DE3) | 15 | recomb. | 38 | 93 |

| | | | | |
|---|---|---|---|---|
| 1. Wet weight, in mg/ml, as determined by harvesting cells by centrifugation and weighing the pellet. 2. Lysozyme added to PopCulture procedure as described in Table 1. 3. Yield in micrograms per ml of culture of β-gal purified using His•Bind Magnetic Agarose Beads, as determined by BCA protein assay. 4. % purity determined by scanning densitometry of Coomassie blue stained SDS polyacrylamide gels. | | | | |

The gel analysis further demonstrates that the overall extraction efficiency was enhanced by lysozyme for β-gal and GST fusion proteins. Again, low level expression of T7 lysozyme in the BL21 (DE3)pLysS host was sufficient to improve target protein extraction efficiency to a level similar to that obtained by treating the BL21(DE3) host with either egg white or recombinant lysozyme. Therefore, when target proteins are expressed in BL21 (DE3)pLysS host strains, maximum PopCulture^{™} extraction efficiency may be obtained without exogenous lysozyme addition.

Effect of culture medium: As shown in Table 3, PopCulture Reagent was equally effective for extraction of proteins expressed in *E. coli* cultured in three standard media formulations. For this experiment, BL21(DE3) containing pET-41b(+) was grown in Terrific Broth (TB), 2X YT, and Luria Broth (LB, which was also used for all other experiments). The ex-pressed His•Tag® GST fusion protein was extracted and purified using PopCulture and His•Bind® Magnetic Agarose Beads. Protein purity was similar for all media tested. However, as expected, cell mass and total protein yield were greater in the richer TB medium.

**Table 3. PopCulture purification of His·Tag GST using different media**

| Medium | Cell Mass¹ | Yield ² | Purity³ |
|---|---|---|---|
| Terrific Broth | 13 | 81 | 90 |
| 2X YT | 11 | 30 | 95 |
| Luria Broth (LB) | 9 | 40 | 89 |

| | | | |
|---|---|---|---|
| 1. Wet weight, in mg/ml, as determined by harvesting cells by centrifugation and weighing the pellet. 2. Yield in micrograms per ml of culture of target protein purified using His·Bind Magnetic Agarose Beads, as determined by BCA protein assay. 3. Determined by scanning densitometry of Coomassie blue stained SDS polyacrylamide gels. | | | |

### EXAMPLE 2

### Fractionation of Positively Charged Proteins by In-media Lysis and Cation Exchange Adsorption or Negatively Charged Proteins by In-media Lysis and Anion Exchange Adsorption

This process of culturing cells in liquid media under condition for endogenous or recombinant target protein production, inducing the culture if necessary to initiate target protein expression, adding concentrated lysis reagent to break the cells and adding capture resin to isolate the target protein(s) from spent culture media and unwanted cellular components, may be broadly applied to fractionate proteins according to charge characteristics. This is accomplished by adding a buffer component to the capture reaction so as to impart a positive or negative charge on the protein(s) of interest. Therefore, proteins with acidic isoelectric points (pI) will be predominantly negatively charged if the pH of the capture reaction is above their pI. These negatively charged proteins are adsorbed to the positively charged anion exchange resin added as a 50% slurry in equilibration buffer, mixed, and reacted 15 min, room temperature, on mixer. Negatively charged proteins on the capture resin are separated from culture media and unwanted cellular components by filtration. Protein-loaded capture resin is washed by mixing with 10-20 resin volumes wash buffer, and isolating the resin by filtration to remove unadsorbed contaminants. Target proteins are eluted using the appropriate elution buffer with high ionic strength or pH change sufficient to desorb the target proteins. This same process could be used for proteins with basic pI employing a lower pH capture reaction and a cation exchange resin. Two dimensional gel analysis of protein samples obtained through these procedures would reveal a population of proteins enriched for those with isoelectric points below the pH of the isolation buffer in the case of anion exchange, and isoelectric points above the pH of the isolation buffer in the case of cation exchange.

### EXAMPLE 3

### Automated Purification of Recombinant Proteins, Effects of Lysozyme, and Protein Level and Activity Measurement

### Methods

MultiPROBE® II HT EX: The Packard-brand MultiPROBE II from PerkinElmer Life Sciences (Downers Grove, IL) is a flexible liquid handling workstation specially designed for the efficient automation of sample preparation procedures utilized in pharmaceutical, biotech, research and clinical applications. Available in 4- and 8-tip models, MultiPROBE II Systems enable dispensing into tubes, vials and microplates using volumes as low as 100 nl. PerkinElmer's patented VersaTip^{™} Plus probe design enables the MultiPROBE II to switch between fixed and disposable tips in one assay. The system's user-friendly WinPREP® software can be optimized for a wide variety of applications, including nucleic acid purification, sequencing reaction setup, PCR setup and clean up, protein purification, automated in-gel digestion, MALDI target spotting, cherry picking, dilutions, Caco-2 screening, and Solid Phase Extractions (SPE).

PerkinElmer's Packard-brand Gripper^{™} Integration Platform expands the capability of MultiPROBE® II EX expanded deck systems, providing an integrated gripper tool capable of "picking-and-placing" SBS-approved microplates, microplate lids, deep-well plates, extraction blocks and selected vacuum manifolds around the deck of MultiPROBE II EX systems. The Gripper also travels beyond the system's right expansion module, enabling integration with approved off-the-shelf devices, such as mixers, incubators, thermal cyclers, hotels, readers, shakers and washers. A full line of application oriented accessories such as automated temperature control of plates and reagents, automated shaker, and automated vacuum control are available to optimize the MultiPROBE II platform and enhance performance of specific applications.

Robotic processing protocol: Cells were cultured in 1.0 ml x 96 wells using a deep-well plate under conditions for target protein production. 0.1 ml PopCulture Reagent^{™} (Novagen, Inc., Madison, WI) containing 25 U Benzonase® Nuclease and 40 U rLysozyme^{™} Solution (Novagen, Inc., Madison, WI) was added to each well, mixed, and incubated for 10 min at room temperature. Optionally, a 1 µl sample was taken from each well for screening expression levels of S•Tag^{™} (Novagen, Inc., Madison, WI) fusion proteins using the FRETWorks^{™} S•Tag Assay (Novagen, Inc., Madison, WI). Equilibrated His•Mag^{™} (Novagen, Inc., Madison, WI) or GST•Mag^{™} Agarose Beads (Novagen, Inc., Madison, WI) was added, mixed, and incubated for 5 min at room temperature. The beads were separated from the extract with the Magnetight^{™} HT96^{™} Stand (Novagen, Inc., Madison, WI) and the supernatant was removed. The beads were washed 2 times by resuspending in 750 µl wash buffer, placing on the magnetic stand, and removing the supernatant from each well. The target protein was eluted by resuspending the beads in the appropriate elution buffer. The beads were collected with the magnetic stand and the supernatant containing the target protein was transferred to a collection plate.

Automated purification using the RoboPop His·Mag and GST·Mag Purification Kits (Novagen, Inc., Madison, WI): Separate cultures of *E*. *coli* strain BL21 (DE3) containing pET-41b(+) and pET-28b(+) β-gal were prepared and protein expression was induced with 1 mM IPTG for approximately 3 h at 30°C. The final cultures had OD600 readings between 4 and 8. The cultures were dis-pensed (1 ml/well) into alternate rows of 2 ml 96-well plates and 100 µl PopCulture^{™} Reagent (Novagen, Inc., Madison, WI) containing 40 units rLysozyme^{™} and 25 units Benzonase® was added to each well. Plates were allowed to react with mixing for 10 min at room temperature (RT). His·Mag or GST·Mag Agarose Beads (Novagen, Inc., Madison, WI) were washed and equilibrated as a 50% slurry with 1X His·Bind® Buffer or 1X GST•Bind^{™} Bind/Wash Buffer (Novagen, Inc., Madison, WI). The equilibrated beads were added to each lysis reaction, mixed, and allowed to react with mixing for 10 min at room temperature. The entire mixture was subjected to a magnetic field using the Magnetight^{™} HT96^{™} Stand to isolate the target-loaded beads. Spent culture media and cellular contaminants were removed with the supernatant while the beads were held by the magnetic field. The beads were washed twice with 750 µl 0.5X His·Bind Wash Buffer or GST Bind/Wash buffer (Novagen, Inc., Madison, WI). The washes were accomplished by removing the plate from the magnetic field, resuspending the beads in wash buffer by shaking on a platform shaker, re-isolating the beads with the magnetic field, and pipetting to remove the supernatant. The purified pro-teins were eluted from the beads with 2 x 150 µl 0.5X His·Bind Elute Buffer or GST•Bind Elute Buffer (Novagen, Inc., Madison, WI). The entire purification process after cell culture and induction was performed automatically by the MultiPROBE II. Samples (2 µg protein) were analyzed by SDS-PAGE (10-20% gradient gel) and Coomassie blue staining. Protein assays were performed by the Bradford method and purity determined by densitometry of the gel scan.

Effect of rLysozyme and Benzonase on protein yield with the RoboPop His·Mag automated protocol: Separate cultures of *E*. *coli* strain BL21(DE3) containing pET-41b(+) and pET-28b(+) β-gal were prepared and protein expression was induced with 1 mM IPTG for approximately 3 h at 30°C. The final cultures had OD600 readings between 4 and 8. The cultures were dispensed (1 ml/well) into alternate rows of a 2 ml 96-well plate and 100 µl PopCulture™ Reagent was added to each well. For the wells into which lysozyme was added, the PopCulture Reagent was pre-mixed with 40 units rLysozyme and/or 25 units Benzonase prior to addition. His•Mag^{™} Agarose Beads were added and the plate was processed using the MultiPROBE II robot. Samples (10 µl eluates) were analyzed by SDS-PAGE (10-20% gradient gel) and Coomassie blue staining. Protein yield and purity were determined by densitometry of the gel scan.

Time course of induction of S•Tag GST with FRETWorks S•Tag Assay: Separate cultures of *E*. *coli* strain BL21(DE3) containing pET-41b(+) (for expression of S•Tag GST) and pET-28b(+) b-gal (as a negative control lacking an S·Tag sequence) were grown in liquid culture and induced with 1 mM IPTG. At the indicated times, 1 ml samples were placed into sequential rows of a 96-well deep well culture plate and 100 µl of PopCulture Reagent containing 40 units of rLysozyme and 25 units of Benzonase Nuclease were added. After mixing for 10 min at room temperature, the crude extracts were used for SDS-PAGE analysis and diluted 1:2500 for the FRETWorks S·Tag Assay according to the standard protocol (20 µl of diluted sample were used per assay). The S·Tag GST fusion protein in the crude extracts was quantified based on a standard curve with known amounts of S·Tag Standard.

Protein yield and purity from 96-well cultures: Separate cultures of *E*. *coli* strain BL21(DE3) containing pET-41b(+) and pET-28b(+) β-gal were grown in alternate rows of a RoboPop Culture Plate by inoculating isolated colonies from LB agar + 34 µg/ml kanamycin plates grown overnight at 37°C into 100 µl TB + phosphates + 0.5% glucose placed in the wells. The inoculated plate was incubated at 24°C with shaking at 300 rpm approximately 16 h. After adding 1.0 ml of the same media the cells were incubated at 30°C with shaking for an additional 1.5 h to an OD600 of 1.0 and induced with 1 mM IPTG for approximately 3 h at 30°C. The final cultures had OD600 readings between 3.5 and 5. For purification, the plates were processed using the RoboPop His·Mag protocol as described above. Samples (2 µg) were analyzed by SDS-PAGE (10-20% gradient gel) and Coomassie blue staining. Protein assays were performed by the Bradford method and purity determined by densitometry of the gel scan.

Expression level solubility screening employing filtration and FRETWorks S-Tag Assay: Separate cultures of *E*. *coli* strain BL21(DE3) containing pET-43.1a(+) (for expression of NusA S-peptide fusion protein) and BL21(DE3)pLacI containing a pTriEx-2 recombinant (for expression of GUS S-peptide fusion protein) were grown in liquid culture and induced with 1mM IPTG for approximately 3 hrs at 30°C. The final cultures had absorbances at 600 nm of 4 to 8. The cultures were dispensed (1 ml/well) into alternate rows of a 2 ml 96-well plates and 100 µl of PopCulture^{™} Reagent (Novagen, Inc., Madison, WI) containing 40 units of rLysozyme^{™} and 25 units of Benzonase® nuclease was added to each well. After mixing 10 min at room temperature, the crude extracts were sampled (200 µl) and processed by filtration (0.45 µm) using the MultiPROBE II robot. The soluble filtrate fraction was collected and a sample diluted 1:2000. The insoluble retentate fraction was solubilized with solubilization reagent, collected, and a sample diluted 1:2000. These dilutions were analyzed by the SDS-PAGE and the FRETWorks assay (5).

### Results:

Purification of fusion proteins by the RoboPop^{™} His•Mag^{™} and GST•Mag^{™} protocol: As test vectors for *E*. *coli* extraction and purification, we used pET-41b(+) for expression of a 35.6 kDa GST•Tag^{™}/His•Tag®/S•Tag^{™} fusion protein and pET-28b(+) for expression of a 119 kDa His•Tag/T7•Tag® β-galactosidase fusion protein. Both fusion proteins can be purified by immobilized metal chelation chromatography (IMAC) using His·Mag Agarose Beads. The 35.6 kDa fusion protein can also be purified using GST•Mag Agarose Beads and also contains the S•Tag peptide, which enables rapid quantification of expression by the homogeneous FRETWorks^{™} S•Tag Assay (5).

The purification results demonstrate the effectiveness of the RoboPop methods with an average yield of 53 µg/ml culture and purity greater than 92% when proteins were purified by the His·Mag method. Yields for purification by the GST·Mag protocol were not as high, but purity was excellent at greater than 98%. Both β-gal and GST purified by these methods were enzymatically active. The reproducibility, absence of degradation products, and lack of cross contamination is seen in the SDS-PAGE analysis of His·Tag β-gal and His·Tag GST purified simultaneously from cultures in alternate rows of the 96-well plate. Although the proteins are purified at ambient temperature and protease inhibitors were not used, no protease degradation was evident. If protease degradation of the target protein is detected, protease inhibitors such as PMSF, AEBSF, Benzamidine or Protease Inhibitor Cocktail Sets III, IV, or V may be added.

Importance of rLysozyme^{™} Solution and Benzonase® Nuclease addition to the robotic protocol: The additions of rLysozyme and Benzonase Nuclease during the extraction stage of the RoboPop^{™} protocol enhanced processing. The combined mechanism of action for these reagents is disruption of the cell membrane and perforation and exposure of the cell wall by the detergent-based PopCulture Reagent, hydrolysis of the N-acetylmuramide linkages in the cell wall by rLysozyme, and complete digestion of the released nucleic acids by Benzonase. β-gal (a tetramer composed of 118 kDa subunits) was not extracted efficiently by PopCulture treatment alone, in contrast to the smaller GST fusion protein (35.6 kDa) (efficiently extracted). The addition of rLysozyme during the PopCulture extraction step did not significantly increase the yield of β-gal and actually decreased the yield of GST by 45%. Treatment with PopCulture plus rLysozyme is required for complete cell lysis, but in the absence of Benzonase, the viscosity resulting from the released nucleic acid interfered with robotic processing. The combination of PopCulture, rLysozyme, and Benzonase synergistically increased the yield of target proteins 40-fold for β-gal and 1.5-fold for GST.

FRETWorks^{™} S•Tag^{™} Assay screening for target protein expression levels: The 15 aa S•Tag peptide enables rapid quantification of fusion proteins by the FRETWorks S•Tag Assay. This ultrasensitive, homogeneous assay is based on the high affinity specific interaction of the S•Tag peptide with S-protein to form active ribonuclease (5), and employs a mixed ribodeoxyribooligonucleotide FRET (fluorescent resonance energy transfer) substrate for RNase containing a fluor on the 5'-end and a quencher on the 3'-end. When cleaved by the ribonuclease S activity of the S•Tag/S-protein complex, quenching is released and a strong fluorescent signal is generated. The FRET substrate appears to be resistant to cleavage by cellular RNases and Benzonase Nuclease, which enables the assay to be used with crude extracts. Fig. 1 shows the FRETWorks Assay results results of a time course of induction of the 36.5 kDa S•Tag GST fusion protein. The FRETWorks Assay results correlated well with SDS-PAGE analysis of the crude extracts prepared by PopCulture/rLysozymeBenzonase treatment. It should be noted that this assay is routinely performed with 20 µl of a 1:2500 dilution of the crude extract and takes less than 10 minutes.

96-Well Cell Culture: In an effort to minimize variability due to culture conditions, the above experiments were performed using aliquots of cultures set up in 50 ml flasks. For true high throughput capability, the entire cell culture process must be carried out in the wells of automation-compatible plates. When RoboPop^{™} His•Mag^{™} purification was conducted using 1 ml cultures set up in a 96-well deep well culture plate, the induced cultures reached a final OD600 between 3 and 3.5, which is about 10-50% lower than obtained using LB broth in 50 ml flasks. The gel analysis and protein purity were very similar to those obtained using flask cultures; however, the yield was slightly lower (32 vs. 40 µg HisTag® β-gal, 45 vs. 67 µg His•Tag GST), which correlates with the decrease in cell mass we observed using these conditions for 96-well culture.

Expression level solubility screening employing filtration and FRETWorks S-Tag Assay: Results from SDS-PAGE showed that GUS S-peptide fusion protein resided in the insoluble fraction and NusA S-peptide fusion protein resided in the soluble fraction. Results of the FRETWorks rapid assay were consistent with the SDS-PAGE analysis.

### EXAMPLE 4

### Centrifugation-Free Extraction and Purification of Recombinant Proteins from Baculovirus Insect Cells

Preparation of Baculoviruses Expressing His·Tag Fusion β-Galactosidase Proteins: The bacterial β-galactosidase gene, LacZ, was cloned into the LIC site of pTriEx-4. Recombinant baculoviruses were generated by cotransfection using BacVector-3000 Triple Cut Virus DNA (Novagen, Inc., Madison, WI) according to Novagen's recommended protocol. For protein expression, Sf9 cells grown in shaker cultures in TriEx Insect Cell Medium (Novagen, Inc., Madison, WI) were infected with baculoviruses at M.O.I. of 5. Infected cells were harvested 72 hours post infection. Half of the infected cells were used for direct in-media cell lysis using Insect PopCulture whereas the other half was processed by the standard method. The standard method employs centrifugation to harvest infected cell pellets while the supernatant is saved and treated as a PopCulture sample and further analyzed for total protein recovery.

Immobilized metal affinity chromatography using Ni-NTA His-Bind Resin (Novagen, Inc., Madison, WI): Standard extractions were performed by resuspending the cell pellet in 1x Cytobuster volume equal to original culture volume. After 15 minutes incubation at room temperature, cell debris was removed by centrifugation. Insect PopCulture extractions were performed by addition of 1/20th culture volume Insect PopCulture reagent (Novagen, Inc., Madison, WI) to the infected cells. To reduce viscosity due to chromosomal DNA, 10 units/ml of Benzonase were added. The mixtures were gently inverted several times and incubated for 15 minutes at room temperature. The lysates were added to equilibrated Ni-NTA His-Bind resin and incubated for 1 hour at 4°C on end-over-end shaker. The lysate/resin mixtures were poured into columns. Unbound and nonspecifically-bound proteins were washed from the columns with 20 column volumes of 50 mM NaH₂PO₄, pH 8.0 containing 20 mM imidazole and 300 mM NaCl. The His·Tag fusion β-galactosidase protein was eluted with 6 column volumes 50 mM NaH₂PO₄, pH 8.0 containing 250 mM imidazole and 300 mMNaCl, and 0.5-ml fractions were collected. Protein concentration was determined by the BCA method. The crude lysate, flow through, and pooled fractions were analyzed by SDS-PAGE.

The SDS-PAGE analysis demonstrates that the Insect PopCulture extraction method combined with Ni-NTA His•BindC® Resin purification produced a nearly homogeneous target protein that was indistinguishable from the protein purified using conventional extraction. The yield data also indicate that the total amount of target protein purified by the Insect PopCulture method was approximately equal to the sum of the protein separately purified from the harvested cell pellet and supernatant fractions (Table 4). The Insect PopCulture method efficiently recovered target protein that had been released into the medium as well as the intracellular target protein. Thus, the Insect PopCulture method results in higher yield through purification and recovery of target protein that has been released into the media due to cell lysis or death as well as the protein extracted from the insect cells.

**Table 4. Purification of His·Tag β-galactosidase from baculovirus insect cell cultures**

| Sample | Purified Protein |
|---|---|
| Cell pellet | 56 µg/ml culture |
| Medium | 64 µg/ml culture |
| Insect PopCulture | 131 µg/ml culture |

### REFERENCES

1. Burgess, R. R., ed. (1987) *Protein Purification: Micro to Macro.* Alan R. Liss Inc., New York.
2. Deutscher, M. P., ed. (1990) *Methods in Enzymology* 182. *Guide to Protein Purification.* Academic Press, Inc., New York.
3. Scopes, R. K. (1994) *Protein Purification: Principles and Practice.* 3^{rd} ed., Springer Verlag, New York.
4. Willson, R.C. in *Manual of Industrial Microbiology and Biotechnology.* Demain, A. L. and Davies, J.L., eds. (1999) 2^{nd} ed., pp. 266-272. ASM Press, Washington, D.C.
5. Raines, R.T., McCormick, M., Van Oosbree, T.R., and Mierendorf, R.C. (2000) *Meth. Enzymol.* 326,362-376.

## Claims

1. A method for recovering a protein from cultured cells comprising:
a) forming a lysis reaction by adding a cell lysis reagent to a cell culture medium without harvesting the cells wherein the cell lysis reagent is a detergent, enzyme, glycoside, or mixture thereof;
b) adding a solid matrix to the lysis reaction to form a mixture, wherein an affinity or adsorptive ligand is attached to the solid matrix and the solid matrix absorbs a protein from the mixture; and
c) separating the solid matrix from the mixture.

2. A method according to claim 1 wherein the solid matrix comprises an affinity resin.

3. A method according to claim 1 wherein the affinity resin comprises magnetic particles.

4. A method according to claim 2 wherein in step c) the affinity resin is separated from the mixture by filtration.

5. A method according to claim 3 wherein in step c) the entire mixture is subjected to a magnetic field to isolate the magnetic particles.

6. A method according to any one of the preceding claims wherein the cell lysis reagent is an enzyme which is a lysozyme, chitinase, glucanase, nuclease, or mixture thereof.

7. A method according to any one of the preceding claims wherein the affinity or adsorptive ligand is Ni-NTA, GST, S-Protein, antibody, or charged functionality.

8. A method according to any one of the preceding claims further comprising eluting a protein from the isolated solid matrix.

9. A method according to any one of the preceding claims wherein the method is automated.

10. A method according to any one of the preceding claims wherein the cells are bacterial, yeast or insect cells. -

11. A method according to any one of the preceding claims wherein the cells are *E. Coli* cells.

## Patentansprüche

1. Verfahren zur Gewinnung eines Proteins aus kultivierten Zellen umfassend:
a) Bilden einer Lysereaktion durch Zugabe eines Lysereagenz' zu einem Zellkulturmedium ohne Ernten der Zellen, wobei das Zell-Lysereagenz ein Detergenz, Enzym, Glycosid oder eine Mischung davon ist;
b) Zugabe einer festen Matrix zur Lysereaktion, um eine Mischung zu bilden, wobei ein Affinitäts- oder Absorptions-Ligand an die feste Matrix angeheftet ist und die feste Matrix ein Protein aus der Mischung absorbiert; und
c) Trennen der festen Matrix von der Mischung.

2. Verfahren nach Anspruch 1, wobei die feste Matrix ein Affinitätsharz umfasst.

3. Verfahren nach Anspruch 1, wobei das Affinitätsharz magnetische Teilchen umfasst.

4. Verfahren nach Anspruch 2, wobei in Schritt c) das Affinitätsharz durch Filtration von der Mischung getrennt wird.

5. Verfahren nach Anspruch 3, wobei in Schritt c) die gesamte Mischung einem magnetischen Feld ausgesetzt wird, um die magnetischen Teilchen zu isolieren.

6. Verfahren nach einem der vorhergehenden Ansprüche, wobei das Zell-Lysereagenz ein Enzym ist, welches ein Lysozym, eine Chitinase, Glucanase, Nuklease oder eine Mischung davon ist.

7. Verfahren nach einem der vorhergehenden Ansprüche, wobei der Affinitäts- oder Absorptions-Ligand Ni-NTA, GST, S-Protein, ein Antikörper oder eine geladene Funktionalität ist.

8. Verfahren nach einem der vorhergehenden Ansprüche, welches außerdem das Eluieren eines Proteins von der isolierten festen Matrix umfasst.

9. Verfahren nach einem der vorhergehenden Ansprüche, wobei das Verfahren automatisiert ist.

10. Verfahren nach einem der vorhergehenden Ansprüche, wobei die Zellen Bakterien-, Hefe- oder Insektenzellen sind.

11. Verfahren nach einem der vorhergehenden Ansprüche, wobei die Zellen E.coli-Zellen sind.

## Revendications

1. Méthode pour la récupération d'une protéine de cellules cultivées comprenant:
a) la formation d'une réaction de lyse par addition d'un réactif de lyse de cellules à un milieu de culture de cellules sans récolter les cellules, où le réactif de lyse de cellules est un détergent, une enzyme, un glycoside, ou un mélange;
b) l'addition d'une matrice solide à la réaction de lyse pour former un mélange, où un ligand par affinité ou d'adsorption est attaché à la matrice solide et la matrice solide absorbe une protéine du mélange; et
c) la séparation de la matrice solide et du mélange.

2. Méthode selon la revendication 1, où la matrice solide comprend une résine d'affinité.

3. Méthode selon la revendication 1, où la résine d'affinité comprend des particules magnétiques.

4. Méthode selon la revendication 2 où à l'étape c) la résine d'affinité est séparée du mélange par filtration.

5. Méthode selon la revendication 3 où à l'étape c) tout le mélange est soumis à un champ magnétique pour isoler les particules magnétiques.

6. Méthode selon l'une quelconque des revendications précédentes, où le réactif de lyse des cellules est une enzyme qui est un lysozyme, une chitinase, une glucanase, une nucléase, ou mélange.

7. Méthode selon l'une quelconque des revendications précédentes où le ligand par affinité ou par adsorption est Ni-NTA, GST, S-Protéine, anticorps, ou une fonctionnalité chargée.

8. Méthode selon l'une quelconque des revendications précédentes comprenant de plus l'élution d'une protéine de la matrice solide isolée.

9. Méthode selon l'une quelconque des revendications précédentes où la méthode est automatisée.

10. Méthode selon l'une quelconque des revendications précédentes où les cellules sont des cellules bactériennes, de levure ou d'insectes.

11. Méthode selon l'une quelconque des revendications précédentes, où les cellules sont des cellules de *E.Coli.*
